# EUROPÄISCHE PATENTANMELDUNG

(11) **EP 2 016 921 A2**
(43) Veröffentlichungstag der Anmeldung: **21.01.2009**
(21) Anmeldenummer: 08075643.0
(22) Anmeldetag: 17.07.2008
(51) Int. Cl.: A61B 19/00

(54) **Stativanordnung und Stativ für ein medizinisch-optisches Instrument**

(30) Priorität: 20.07.2007 DE 102007034286
(71) Anmelder: Carl Zeiss Surgical GmbH, 73447 Oberkochen (DE)
(72) Erfinder: Brenner, Roland, 74599 Wallhausen (DE)
(74) Vertreter: Theobald, Andreas

(57) **Zusammenfassung**

Es wird ein Stativ (1) für ein medizinisch-optisches Instrument (3) mit einer Lenkeranordnung (15) mit wenigstens einem Lenker (13, 17, 19, 21), und einer aktiven Schwingungsdämpfungsvorrichtung (33, 35, 37), welche wenigstens einen Schwingungsaufnehmer (33) zum Aufnehmen einer zu dämpfenden Schwingung und wenigstens einen Aktuator (37) zum Erzeugen einer dämpfenden Gegenschwingung umfasst, zur Verfügung gestellt. Der wenigstens eine Aktuator (37) ist von einem Schwingungsbauch der zu dämpfenden Schwingung entfernt an der Oberfläche eines Lenkers (19) der Lenkeranordnung (15) angeordnet und zum Ausüben einer Kraft auf den Lenker (19) ausgebildet.

## Beschreibung

Die vorliegende Erfindung betrifft ein Stativ für ein medizinisch-optisches Instrument mit einer Lenkeranordnung mit wenigstens einem Lenker und einer aktiven Schwingungsdämpfungsvorrichtung. Daneben betrifft die Erfindung eine Stativanordnung mit einem derartigen Stativ, einer an der Lenkeranordnung angeordneten Halterung für das medizinisch-optische Instrument und einem an der Halterung des Stativs befestigten medizinisch-optischen Instrument.

Beispielsweise ein Stativ für die Neurochirurgie trägt in der Regel ein Operationsmikroskop mit bis zu 30-facher Vergrößerung. Als mechanisches Gebilde ist das Stativ jedoch nur endlich steif und zeigt daher unter Belastung eine gewisse Verformung. Das Stativ unendlich steif zu bauen ist technisch nicht möglich. Zudem bringt eine hohe Steifheit auch ein sehr hohes Eigengewicht mit sich.

Die endliche Steifheit macht das Stativ zu einem schwingfähigen System, d.h. durch Anstoßen oder durch eine kleine äußere periodische Kraft kann das Stativ zu Schwingungen angeregt werden. Schwingt das Stativ, so ist die Bildqualität des Operationsmikroskops erheblich beeinflusst, was insbesondere bei neurochirurgischen Operationen Nachteile mit sich bringt.

Das Anstoßen kann in der Praxis nicht verhindert werden. Es ist jedoch möglich, die Ausschwingzeit des Stativs nach einem Anstoß konstruktiv so klein wie möglich zu halten. Dies geschieht in der Regel durch eine geeignete Dämpfung in den Drehgelenken, die Teil des Stativs sind. Wird das Stativ hingegen nicht lediglich angestoßen, sondern durch eine periodische Kraft angeregt, so kann nur sehr wenig gegen die dadurch hervorgerufenen Schwingungen unternommen werden. Periodische Kräfte können bspw. durch Gebäudeschwingungen, Schwingungen von Klimaanlagen oder durch Lüfter verursacht werden. Insbesondere bei Deckenstativen, die mechanisch fest mit der Decke eines Gebäudes verbunden sind, ist die Anfälligkeit gegen Schwingungen besonders hoch. Dämpfer klassischer Bauart wie etwa Gummipuffer bringen leider wenig und können die Schwingungen aufgrund ihres Übertragungsverhaltens bei periodischer Anregung unter Umständen durch Resonanzen sogar verschlimmern.

Es sind daher Stative vorgeschlagen worden, in denen eine aktive Schwingungsdämpfung insbesondere periodisch angeregte Schwingungen unterdrücken soll.

So beschreiben bspw. die DE 10 2004 004 602 A1 und die DE 10 2004 063 606 A1 Stative und Haltevorrichtungen mit aktiver Schwingungsdämpfung, in denen Regelkreise vorhanden sind, die zur Schwingungsdämpfung auf einen Antrieb des Stativs oder der Haltevorrichtung einwirken.

Aus US 2001/002432 A1 ist ein Mikroskop mit einem dynamischen Dämpfer bekannt, der beim Mikroskop im Bereich des freien Endes eines Haltearms des Stativs angeordnet ist. Die aktive Schwingungsdämpfung erfolgt so an der Stelle, wo die zu dämpfende Schwingung die größte Auslenkung herbeiführt.

In DE 43 245 38 A1 und in DE 43 427 17 A1 sind Mikroskopstative beschrieben, die eine Kräfte ausübende Einrichtung zur aktiven Schwingungsdämpfung beinhalten. Die Kräfte ausübende Einrichtung ist zwischen einem vertikalen Tragarm und einem horizontalen Tragarm bzw. zwischen einem vertikalen Tragarm und einem Operationsmikroskop angeordnet.

In EP 1 447 700 A2 ist ein Mikroskop mit einem Stativ beschrieben, wobei das Mikroskop und/oder das Stativ wenigstens ein tragendes Teil und eine Schwingungsausgleichseinrichtung aufweisen. Als Schwingungsausgleichseinrichtung kommt ein selbstregulierendes Bauelement zum Einsatz, das auf Basis der Messung von Schwingungen integrierte Antriebselemente so ansteuert, dass diese der Schwingung in Istzeit derart entgegenwirken, dass die Schwingung nicht zu einer Lageveränderung der äußeren Konturen bzw. der entscheidenden Schnittstellen an dem Bauteil führen. Derartige Bauelemente sind unter den Begriff ARES-Bauelemente aus dem Artikel von E. J. Breitbach et al.: "Adaptive Structure - Concepts and Prospects" des Institute of Aeroelesticity und des Institute of Structural Mechanics des Deutschen Zentrums für Luft- und Raumfahrt, Seiten 3 bis 8, (Publikationsdatum unbekannt), beschrieben. Hierbei steht ARES für Actively Reacting Flexible Structure. Die ARES-Bauelemente sind in EP 1 447 700 A2 insbesondere an einem potenziellen Auftrittsort von Schwingungsbäuchen angeordnet.

Gegenüber diesem Stand der Technik ist es Aufgabe der vorliegenden Erfindung, ein vorteilhaftes Stativ zum Halten eines medizinisch-optischen Instruments sowie eine vorteilhafte Stativanordnung umfassend ein Stativ, eine Haltervorrichtung für ein medizinisch-optisches Instrument und ein an der Halterung angeordnetes medizinisch-optisches Instrument zur Verfügung zu stellen.

Diese Aufgabe wird durch ein Stativ nach Anspruch 1 bzw. eine Stativanordnung nach Anspruch 12 gelöst. Die abhängigen Ansprüche enthalten vorteilhafte Ausgestaltungen der Erfindung.

Ein erfindungsgemäßes Stativ für ein medizinisch-optisches Instrument, das sowohl als Bodenstativ als auch als Deckenstativ ausgebildet sein kann, umfasst eine Lenkeranordnung mit wenigstens einem Lenker und eine aktive Schwingungsdämpfungsvorrichtung. Die aktive Schwingungsdämpfungsvorrichtung ist mit wenigstens einem Schwingungsaufnehmer zum Aufnehmen einer zu dämpfenden Schwingung und mit wenigstens einem Aktuator zum Erzeugen einer dämpfenden Gegenschwingung ausgestattet. Der wenigstens eine Aktuator ist von einem Schwingungsbauch der zu dämpfenden Schwingung entfernt an der Oberfläche eines Lenkers angeordnet und zum Ausüben einer Kraft auf den Lenker, bspw. auf die Oberfläche des Lenkers, ausgebildet. Hierbei kann die Oberfläche eine äußere Oberfläche des Lenkers oder, bei einem hohlen Lenker, eine innere Oberfläche sein.

Durch das von einem Schwingungsbauch der zu dämpfenden Schwingung entfernte Anordnen des Aktuators ist es möglich, die zu dämpfende Schwingung durch Ausüben einer Kraft auf den Lenker, bspw. auf die Oberfläche des Lenkers, zu dämpfen, ohne dass der Aktuator eine große Auslenkung des Lenkers herbeiführen muss. Dabei wird im Rahmen der Erfindung die Schwingungsfähigkeit des Stativs, die bisher eigentlich als störende Eigenschaft angesehen worden ist, ausgenutzt, um eine geeignete Gegenschwingung am Ort des Schwingungsbauchs, bspw. am freien Ende eines Tragarms, zu erzeugen. Im Unterschied dazu sind im Stand der Technik, in dem die Aktuatoren im Bereich des Schwingungsbauchs, also am Ort der maximalen Auslenkung, angeordnet sind, größere Aktuatorbewegungen nötig, um die Bewegung im Schwingungsbauch auszugleichen.

Mit besonders kleinen Aktuatorbewegungen lassen sich Schwingungen dämpfen, wenn wenigstens ein Aktuator an oder in der Nähe derjenigen Stelle des Lenkers angeordnet ist, an der die auf den Lenker einwirkende Last des medizinisch-optischen Instruments die größte mechanische Spannung an der Oberfläche des Lenkers bzw. das größte Biegemoment im Lenker erzeugt. An dieser Stelle können Aktuatorbewegungen besonders effektiv zur Erzeugung einer Gegenschwingung in den Lenker eingekoppelt werden.

Insbesondere können wenigstens zwei Aktuatoren vorhanden sein, die an zueinander nicht parallelen Oberflächenbereichen des Lenkers angeordnet sind. Diese Ausgestaltung ermöglicht es, Schwingungen in zwei zueinander senkrechten Richtungen aktiv zu dämpfen.

In einer vorteilhaften Ausgestaltung des erfindungsgemäßen Stativs ist der Lenker der Lenkeranordnung ein Tragarm, an dem eine Halterung für das medizinisch-optische Instrument zu befestigen ist. Dieser neigt besonders zu Schwingungen, da in der Regel ein relativ großer Abstand zwischen einem Lagepunkt des Tragarmes und dem freien Ende des Tragarmes, an dem die Halterung zu befestigen ist, vorliegt.

Der Aktuator zum Erzeugen der Gegenschwingung kann insbesondere ein piezoelektrisches Material umfassen. Er kann bspw. ein piezokeramischer Aktuator sein. Piezokeramische Aktuatoren kommen in Piezo-Stapelbauweise, Piezo-Folienbauweise und Piezofaser-Bauweise vor und können auch als solche im erfindungsgemäßen Stativ zum Einsatz kommen. Aufgrund ihrer Flexibilität eignen sich piezokeramische Aktuatoren insbesondere dann, wenn sie in der Piezo-Folienbauweise oder in Piezofaser-Bauweise ausgeführt sind, zum Einsatz im erfindungsgemäßen Stativ.

Alternativ kann das piezoelektrische Material auch ein ferroelektrisches Material, etwa ein ferroelektrischer Kunststoff wie bspw. Polyvinylidenfluorid sein. Insbesondere ferroelektrische Kunststoffe ermöglichen eine weitgehend freie Formgebung für die Aktuatoren und so ein optimales Anpassen der Aktuatorform an die Oberflächengeometrie des Lenkers.

Anstatt eines piezoelektrischen Materials kann der Aktuator aber auch einen magnetischen Masseschwinger mit einer magnetisch zum Schwingen anregbaren Masse und einer elektromagnetischen Anregungseinrichtung zum Anregen einer Schwingung der Masse umfassen. Außer als magnetischer Festkörper kann die zum Schwingen anregbare Masse des Masseschwingers auch als in einem Gefäß befindliche ferromagnetische oder magnetorheologische Flüssigkeit ausgebildet sein. Eine leitfähige magnetische Flüssigkeit, die sich zum Einsatz in einem Aktuator eignet, ist bspw. in US 2007/0114486 A1 beschrieben. Auf diese Druckschrift wird hinsichtlich geeigneter Flüssigkeiten verwiesen. Die Schwingung der Masse kann durch ein geeignet angelegtes elektromagnetisches Feld einfach in ihrer Amplitude, ihrer Frequenz und in ihrer Richtung gesteuert werden. Hierbei bietet insbesondere die Verwendung einer Flüssigkeit als Masseschwinger die Möglichkeit, mit ein und demselben Schwinger unterschiedliche geometrische Schwingungen zu erzeugen. Zudem besitzen ferrofluide eine sehr geringe Hysterese, was für den Steuerprozess vorteilhaft ist.

Der Schwingungsaufnehmer der aktiven Schwingungsdämpfungsvorrichtung kann insbesondere ein am medizinisch-optischen Instrument oder an der Halterung des medizinisch-optischen Instruments zu befestigender Beschleunigungssensor sein. Insbesondere wenn er am Instrument selbst befestigt ist, ermöglicht er ein präzises Ermitteln der tatsächlichen Instrumentenschwingung.

Alternativ ist es jedoch auch möglich, den Schwingungsaufnehmer als Biegesensor oder Spannungssensor auszubilden, der an oder in der Nähe einer Stelle eines Lenkers der Lenkeranordnung angeordnet ist, an der die auf den Lenker einwirkende Last des medizinisch-optischen Instruments die größte elastische Verformung und damit die größte mechanische Oberflächenspannung und das größte Biegemoment erzeugt. An dieser Stelle erzeugt nämlich auch eine Schwingung eine deutlich messbare Verformung, die mittels des Biegesensors bzw. des Spannungssensors einfach zu ermitteln ist. Als Biege- oder Spannungssensoren kommen hierbei bspw. piezoelektrische oder ferroelektrische Elemente in Betracht, die grundsätzlich nach dem gleichen Prinzip wie ein piezoelektrische Aktuator oder ein ferroelektrische Aktuator funktionieren und auch einen entsprechenden Aufbau besitzen können. Während beim Aktuator eine Verformung durch Anlegen eines elektrischen Feldes herbeigeführt wird, wird beim Biege- oder Spannungssensor der umgekehrte Prozess genutzt, nämlich, dass eine Verformung des piezoelektrischen oder ferroelektrischen Elements zu einer messbaren Spannung führt, welche ein Ermitteln der Schwingung ermöglicht.

Eine erfindungsgemäße Stativanordnung umfasst ein erfindungsgemäßes Stativ, eine an der Lenkanordnung angeordnete Halterung für das medizinisch-optische Instrument und ein an der Halterung befestigtes medizinisch-optisches Instrument, welches insbesondere ein Operationsmikroskop sein kann. Aufgrund der mit Bezug auf das Stativ beschriebenen vorteilhaften aktiven Schwingungsdämpfung ermöglicht das erfindungsgemäße Stativsystem ein Arbeiten mit dem medizinisch-optischen Instrument ohne störende Schwingungen. Dabei kann der Aktuator auch an einem Lenker der Halterung angeordnet sein, der in diesem Zusammenhang auch als Teil des Stativs und dessen Lenkeranordnung angesehen werden kann, auch wenn die Halterung in der Regel ein eigenständiges Bauteil ist. Wenn mit Bezug auf das erfindungsgemäße Stativ von einem Lenker der Lenkeranordnung die Rede ist, soll daher darunter auch ein Lenker einer eventuell an Stativ befestigten Halterung zu verstehen sein.

Weitere Merkmale, Eigenschaften und Vorteile der vorliegenden Erfindung ergeben sich aus der nachfolgenden Beschreibung von Ausführungsbeispielen unter Bezugnahme auf die beiliegenden Figuren.
Figur 1 zeigt ein erstes Ausführungsbeispiel für ein Stativsystem mit einem erfindungsgemäßen Stativ.
Figur 2 zeigt ein zweites Ausführungsbeispiel für ein Stativsystem mit einem erfindungsgemäßen Stativ.
Figur 3 zeigt ein drittes Ausführungsbeispiel für ein Stativsystem mit einem erfindungsgemäßen Stativ.
Figur 4 zeigt ein Detail aus Figur 3.
Figur 5 zeigt ein viertes Ausführungsbeispiel für ein Stativsystem mit einem erfindungsgemäßen Stativ.
Figur 6 zeigt ein fünftes Ausführungsbeispiel für ein Stativsystem mit einem erfindungsgemäßen Stativ.
Figur 7 zeigt den Verlauf von Oberflächenspannungen in einem am freien Ende belasteten Lenker.

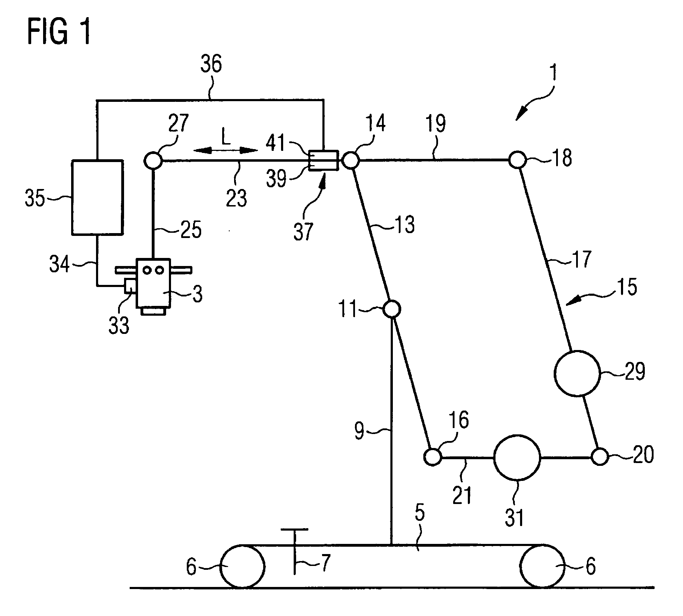

Ein erstes Ausführungsbeispiel für ein Stativsystem mit einem erfindungsgemäßen Stativ 1 und einem Stereo-Operationsmikroskop 3 als medizinisch-optischem Instrument ist stark schematisiert in Figur 1 dargestellt. Das Stativ 1 ruht auf einem Stativfuß 5, an dessen Unterseite Rollen 6 vorhanden sind, die ein Verfahren des Stativs ermöglichen. Um ein ungewolltes Verfahren zu Verhindern, besitzt der Stativfuß 5 außerdem eine Fußbremse 7. Eine Stativsäule 9 (oder eine Konsole) ist auf dem Stativfuß 5 angeordnet und weist ein Gelenk 11 auf, an dem ein erster Lenker 13 einer Lenkeranordnung 15 schwenkbeweglich befestigt ist. Zudem weist auch die Stativsäule 9 ein in der Figur nicht dargestelltes Drehgelenk auf.

An den beiden entgegengesetzten Enden des ersten Lenkers 13 sind Gelenke 14, 16 vorhanden, über die ein erster Querlenker 19 bzw. ein zweiter Querlenker 21 mit dem ersten Lenker 13 schwenkbeweglich verbunden sind. Ein zweiter Lenker 17 ist über Gelenke 18, 20, die sich im Wesentlichen an seinen beiden entgegengesetzten Enden befinden, mit dem ersten Querlenker 19 bzw. dem zweiten Querlenker 21 derart verbunden, dass eine Gelenkanordnung 15 in Form eines Parallelgestänges entsteht.

Der erste Querlenker 19 ist gegenüber dem zweiten Querlenker 21 verlängert und steht über das Parallelgestänge vor. Das Gelenk 14 kann dabei als Lager bzw. Lagergelenk des vorstehenden Abschnitts angesehen werden. Am freien Ende des vorstehenden Abschnittes 23 des Querlenkers 19 ist eine Mikroskophalterung 25 über ein Gelenk 27 dreh- und schwenkbeweglich angeordnet. Die Mikroskophalterung 25 kann entweder ein eigenständiges Bauteil oder ein integraler Bestandteil des Stativs 1 sein. An der Mikroskophalterung 25 ist das Stereo-Operationsmikroskop 3 befestigt.

Zum Ausgleichen des Gewichts des Operationsmikroskops 3 sind am zweiten Lenker 17 sowie am zweiten Querlenker 21 Ausgleichsgewichte 29, 31 angeordnet, die dafür sorgen, dass keine resultierenden Drehmomente aufgrund des Gewichts des Operationsmikroskops 3 bestehen bleiben. Zum Bewegen des Mikroskops sind daher im Grunde keine Motoren und Bremsen nötig. Dennoch können in den einzelnen Gelenken Elektromotoren vorhanden sein, um ein motorgetriebenes Bewegen des Stativs 1 zu ermöglichen. Außerdem können die Gelenke Bremsen aufweisen, mit denen sie gegen ein ungewolltes Bewegen gesichert werden können.

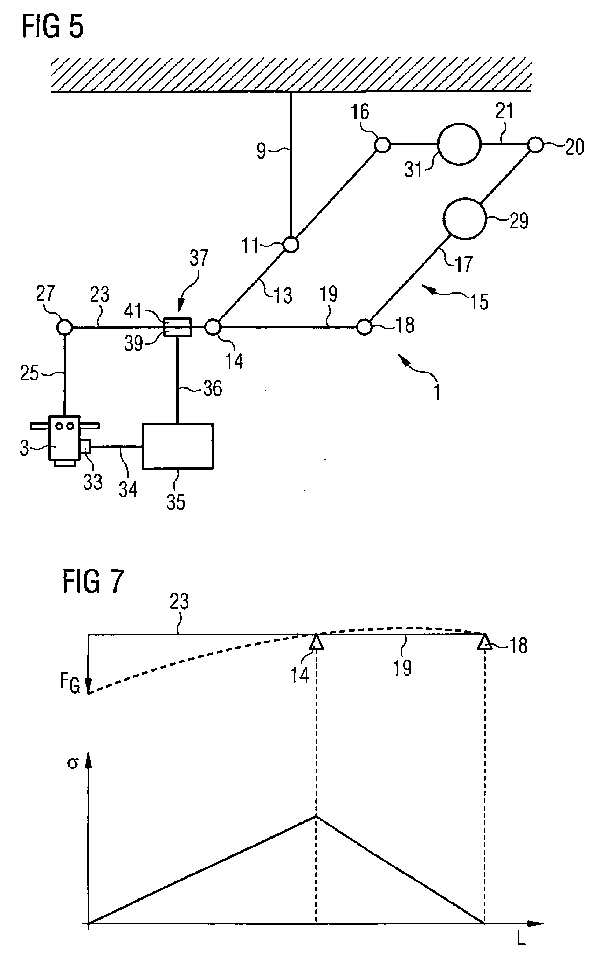

Über die Räder 6 und die Bremse 7 können bspw. Gebäudeschwingungen auf das Stativ 1 übertragen werden, die zu einem Schwingen des vorstehenden Abschnittes 23 des als Tragarm ausgebildeten ersten Querlenkers 19 führen. Als schwingende Masse ist hierbei das Operationsmikroskop 3 zusammen mit der Mikroskophalterung 25 anzusehen. Die Schwingung führt zu einer Auslenkung im Bereich des Gelenks 27, welche wiederum mechanische Spannungen in der Oberfläche des Lenkers 19 induziert. Der Spannungsverlauf, der von der Gewichtskraft des Operationsmikroskops 3 und der Halterung 25 im ersten Querlenker 19 induziert wird, ist in Figur 7 skizziert. Die Spannung σ steigt vom Gelenk 27 ausgehend in Richtung auf das Lagergelenk 14 linear an und erreicht am Lagergelenk 14 ihren maximalen Wert. Von Lagergelenk 14 ausgehend nimmt sie dann zum Gelenk 18 hin wieder linear ab. Liegt nun eine Schwingung des ersten Querlenkers 19 vor, so führt dies dazu, dass die auf das freie Ende des Lenkers 19 einwirkende Gewichtskraft periodisch durch eine Kraft überlagert wird, die aus der durch die Schwingung erzeugten Beschleunigung des Mikroskops 3 resultiert. Dies macht sich durch eine periodische Variation der Spannungen σ in der Oberfläche des ersten Lenkers 19 bemerkbar.

Um auf das Mikroskop 3 übertragene Schwingungen dämpfen zu können, ist das Stativ 1 mit einem aktiven Schwingungsdämpfungssystem ausgestattet. Dieses umfasst einen Schwingungsaufnehmer 33, der im vorliegenden Ausführungsbeispiel als ein am Operationsmikroskop 3 anzubringender Beschleunigungssensor ausgestaltet ist. Weiterhin umfasst die Vorrichtung einen Regler 35 sowie eine Aktuatoranordnung 37. Eine vom Beschleunigungssensor 33 aufgrund einer Schwingung des Operationsmikroskops 3 erfasste Beschleunigung wird in ein Beschleunigungssignal umgewandelt und über eine Signalleitung 34 an den Regler 35 weitergeleitet. Dort wird mittels eines geeigneten Regelalgorithmus auf der Basis des Beschleunigungssignals eine zu erzeugende Gegenschwingung ermittelt, die in der Lage ist, die Schwingung des Operationsmikroskops 3 durch destruktive Interferenz auszulöschen. Auf der Basis der Gegenschwingung wird von Regler 35 ein Stellsignal über die Signalleitung 36 an die Aktuatoranordnung 37 ausgegeben. Das Stellsignal veranlasst die Aktuatoranordnung, die vom Regler 35 ermittelte Gegenschwingung im ersten Querlenker 19 zu erzeugen.

Im vorliegenden Ausführungsbeispiel umfasst die Aktuatoranordnung 37 zwei piezokeramische Elemente 39, 41, die als Piezofaser-Elemente ausgebildet sind. Eines der Piezofaser-Elemente 39 ist an der Unterseite des ersten Querträgers 19 unmittelbar vor dem Lagergelenk 14 angeordnet. Das zweite Piezofaser-Element 41 ist ebenfalls unmittelbar vor dem Lagergelenk 14 auf der Oberseite des ersten Querlenkers 19 angeordnet. Beide Piezofaser-Elemente 39, 41 sind fest mit der Oberfläche des ersten Querträgers 19 verbunden und können somit durch Kontraktion und Expansion in Längsrichtung L des ersten Querlenkers 19 Spannungen in dessen Oberfläche induzieren. Wenn nun eine mechanische Spannung aufgrund der Mikroskopschwingung in der Oberfläche vorliegt, die zu einer Ausdehnung im Bereich des oberen Piezofaser-Elements 41 führt, kann dieser Spannung durch eine Kontraktion dieses Piezofaser-Elements 41 entgegengewirkt werden, so dass sie sich aufheben lässt. Wenn dieses Entgegenwirken mit der Frequenz der zu dämpfenden Schwingung erfolgt, entsteht eine destruktive Indifferenz, die zur Auslöschung der zu dämpfenden Schwingung führt.

Obwohl im vorliegenden Ausführungsbeispiel zwei Piezofaser-Elemente 39, 41 vorhanden sind, reicht es grundsätzlich aus, ein Piezofaser-Element vorzusehen, sofern das Piezofaser-Element sowohl eine Expansion als auch eine Kontraktion zulässt. Falls das Piezofaser-Element jedoch lediglich so ausgestaltet ist, dass es entweder eine Kontraktion oder eine Expansion, aber nicht beides ausführen kann, so können Piezofaser-Elemente wie dargestellt an entgegengesetzten Oberflächen des Lenkers 19 angeordnet sein. Je nachdem, ob die Schwingung gerade zu einer Auslenkung des ersten Querträgers nach oben oder nach unten führt, würde dann wenigstens eines der beiden Piezofaser-Elemente zum Kompensieren der Auslenkung aktiviert.

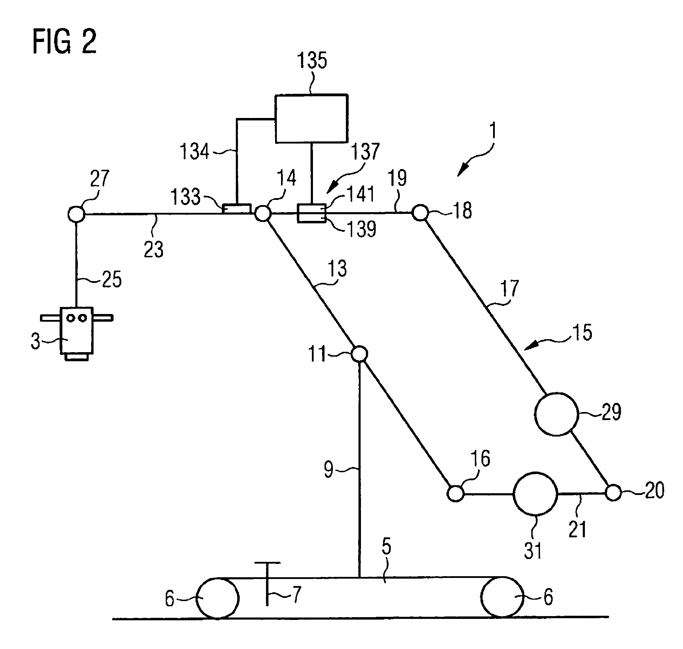

Ein zweites Ausführungsbeispiel für das erfindungsgemäße Stativ ist in Figur 2 dargestellt. Der mechanische Aufbau des Stativs unterscheidet sich nicht von dem mechanischen Aufbau des in Figur 1 gezeigten Stativs und wird daher nicht noch einmal beschrieben, um Wiederholungen zu vermeiden.

Der Unterscheid des in Figur 2 gezeigten Stativs zum in Figur 1 gezeigten Stativ liegt alleine in der Schwingungsdämpfungsvorrichtung. Die Schwingungsdämpfungsvorrichtung umfasst im zweiten Ausführungsbeispiel einen Schwingungsaufnehmer 133, der als Biegesensor ausgebildet ist, einen Regler 135 sowie eine Aktuatoranordnung 137. Der Regler 135 ist über eine Signalleitung 134 zum Empfang eines Biegesignals mit dem Biegesensor 133 und zur Ausgabe eines Stellsignals über eine Signalleitung 136 mit der Aktuatoranordnung 137 verbunden. Das Biegesignal repräsentiert hierbei ein durch die zu dämpfende Schwingung verursachtes Biegemoment.

Als Biegesensor 133 kann ein piezoelektrisches Element zum Einsatz kommen, bspw. eine piezoelektrische Keramik. Diese ist so ausgestaltet, dass sie eine elektrische Spannung erzeugt, wenn sie gebogen wird, wobei der Betrag der Spannung vom Ausmaß der Biegung des piezoelektrischen Sensors 133 abhängt. Das aufgrund einer am freien Ende des ersten Querträgers angreifenden Kraft entstehende Biegemoment zeigt einen Verlauf, der dem in Figur 7 dargestellten Verlauf der Oberflächenspannungen σ entspricht, d.h. ein Maximum am Lagergelenk 14 aufweist. Eine Schwingung des Operationsmikroskops 3 erzeugt daher im Bereich des Lagergelenks 14 ein maximales Biegemoment, das sich gut zum Detektieren einer Schwingung eignet.

Auf der Basis des detektierten Biegemomentes berechnet der Regler 135 eine geeignete Gegenschwingung und gibt ein die Gegenschwingung repräsentierendes Stellsignal an die Aktuatoranordnung 137 aus.

Im vorliegenden Ausführungsbeispiel ist die Aktuatoranordnung 137 in Form zweier Stapel-Piezoelemente 139, 141 ausgeführt. Diese sind so aufgebaut, dass sie bei Anlegen einer geeigneten Spannung ein Biegemoment im ersten Querlenker 19 erzeugen, dass dem von der zu dämpfenden Schwingung erzeugten Biegemoment entgegengesetzt ist. Auf diese Weise kann die Gegenschwingung in den ersten Querlenker 19 eingebracht werden, so dass die zu dämpfende Schwingung durch destruktive Interferenz aufgehoben wird. Statt hintereinander wie im vorliegenden Ausführungsbeispiel können der Biegesensor und die Piezoelemente des Aktuators auch quer zur Längsrichtung des Lenkers 19 nebeneinander angeordnet sein.

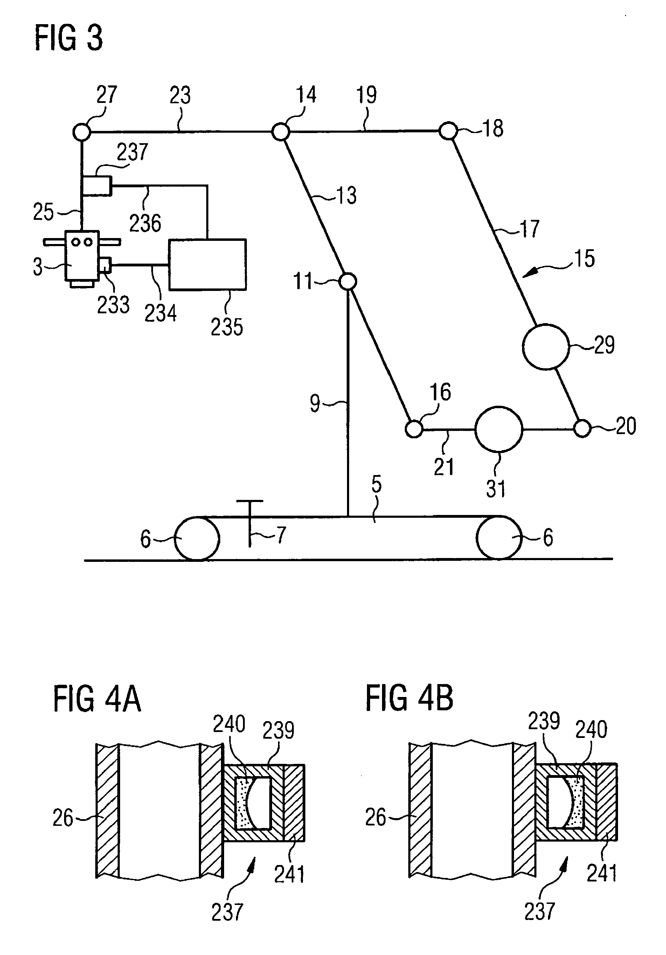

Figur 3 zeigt ein drittes Ausführungsbeispiel für eine Stativvorrichtung mit einem erfindungsgemäßen Stativ 1. In seinem mechanischem Aufbau entspricht das Stativ 1 dem der beiden vorausgegangenen Ausführungsbeispiele. Seine mechanischen Elemente sind daher mit denselben Bezugsziffern wie in diesen beiden Ausführungsbeispielen bezeichnet und werden nicht noch einmal erläutert, um Wiederholungen zu vermeiden.

Das dritte Ausführungsbeispiel unterscheidet sich von den beiden anderen Ausführungsbeispielen durch seine aktive Schwingungsdämpfungsvorrichtung. Diese umfasst einen am Operationsmikroskop 3 anzubringenden Schwingungsaufnehmer 233, einen Regler 235 sowie einen Aktuator 237, der im vorliegenden Ausführungsbeispiel nicht am den Tragarm repräsentierenden ersten Querlenker 19 angeordnet ist, sondern an einem Lenker der Mikroskophalterung 25. Der Schwingungsaufnehmer 233 ist wie im ersten Ausführungsbeispiel als Beschleunigungssensor ausgebildet und über eine Signalleitung 234 zum Ausgeben eines die Mikroskopbeschleunigung repräsentierenden Beschleunigungssignals mit dem Regler 235 verbunden. Außerdem ist der Regler über eine Signalleitung 236 zum Ausgeben eines Stellsignals mit dem Aktuator 237 verbunden.

Der Regler 235 enthält einen Regelalgorithmus, der auf der Basis der empfangenen Beschleunigungsdaten eine geeignete Gegenschwingung zum Dämpfen der Mikroskopschwingung ermittelt und ein geeignetes Stellsignal zum Erzeugen der Gegenschwingung an den Aktuator 237 ausgibt. Der Aktuator 237 erzeugt dann die Gegenschwingung, die zu einer destruktiven Interferenz mit der zu dämpfenden Schwingung führt, als Reaktion auf das Stellsignal.

Im vorliegenden Ausführungsbeispiel findet als Aktuator 237 ein Masseschwinger Anwendung, der eine zum Schwingen anregbare magnetische Masse sowie eine elektromagnetische Anregungseinrichtung umfasst, mit der die magnetische Masse zum Schwingen angeregt werden kann. Der Masseschwinger ist in den Figuren 4a und 4b schematisch dargestellt. Die Figuren zeigen einen Ausschnitt aus einem hohlen Lenker 26 der Mikroskophalterung 25, an dessen Außenseite der Masseschwinger 237 befestigt ist. Er kann aber alternativ auch im Inneren des Lenkers 26 angeordnet sein. Der Masseschwinger 237 umfasst einen Behälter 239, in dem sich eine ferromagnetische Flüssigkeit 240 befindet und der am Lenker 26 befestigt ist. An der dem Lenker 26 abgewandten Seite des Behälters 239 ist ein Elektromagnet 241 befestigt, mit dem sich ein magnetisches Wechselfeld erzeugen lässt. Je nach Polarisierung des magnetischen Wechselfeldes wird die ferroelektrische Flüssigkeit 240 entweder in Richtung auf den Lenker 26 gedrückt (Figur 4A) oder in Richtung auf den Elektromagneten 241 (Figur 4B). Mit Hilfe des magnetischen Wechselfeldes kann so einen Schwingung der Flüssigkeit 240 im Behälter 239 herbeigeführt werden, die sich auf den Lenker 26 überträgt. Die Steuerung des Elektromagneten erfolgt so durch den Regler 235, dass die im Lenker 26 induzierte Schwingung die zu dämpfende Schwingung des Operationsmikroskops 3 durch destruktive Interferenz aufhebt. Statt einer ferroelektrischen Flüssigkeit kann auch eine magnetorheologische Flüssigkeit Verwendung finden, die ebenfalls auf Magnetfelder reagiert, dabei jedoch ihre Viskosität ändert. Eine ferroelektrische Flüssigkeit ändert dagegen ihre Viskosität bei Anliegen eines Magnetfeldes nicht.

Ein viertes Ausführungsbeispiel für die erfindungsgemäße Stativvorrichtung ist in Figur 5 dargestellt. Dieses Ausführungsbeispiel entspricht dem mit Bezug auf Figur 1 beschriebenen Ausführungsbeispiel mit dem Unterschied, dass es sich bei dem Stativ 1 nicht um ein Bodenstativ wie im ersten Ausführungsbeispiel, sondern um ein Deckenstativ handelt. Statt auf einem Stativfuß ist die Stativsäule 9 an der Decke hängend angeordnet. Unerwünschte Schwingungen können bspw. über die Decke in dieses Stativ 1 eingekoppelt werden. Die aktive Schwingungsdämpfungsvorrichtung entspricht vollständig der des ersten Ausführungsbeispiels.

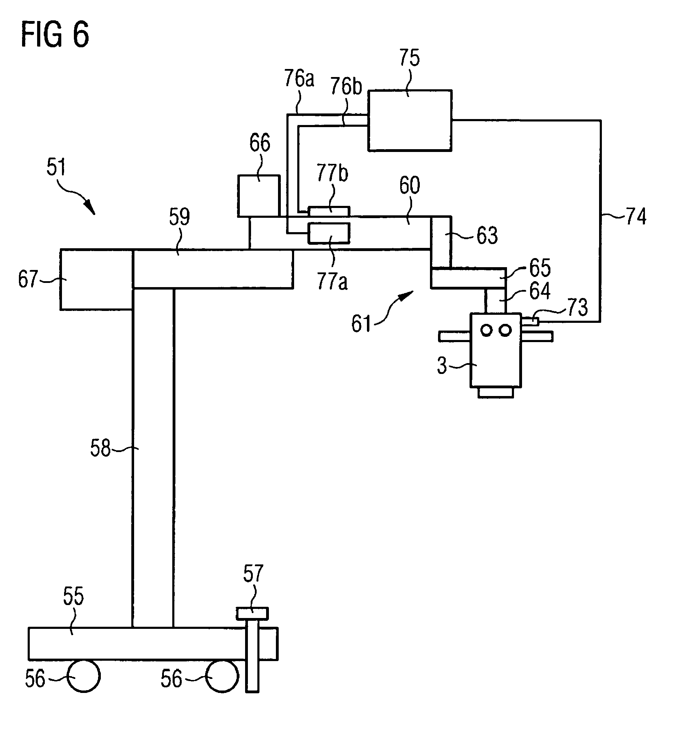

Ein fünftes Ausführungsbeispiel für eine Stativvorrichtung mit einem erfindungsgemäßen Stativ ist in Figur 6 dargestellt. Bei dem Stativ 51 handelt es sich um ein fahrbares Motorstativ mit erhöhter Stabilität. Es umfasst einen Stativfuß 55, an dessen Unterseite Rollen 56 vorhanden sind, die ein Verfahren des Stativs 51 ermöglichen. Um ein ungewolltes Verfahren des Stativs 51 zu verhindern, besitzt der Stativfuß 55 außerdem eine Fußbremse 57. Das Stativ umfasst weiterhin als Stativglieder eine höhenverstellbare Stativsäule 58, einen Tragarm 59, einen Federarm 60 und eine Mikroskopaufhängung bzw. -halterung 61, welche ihrerseits ein Verbindungselement 63, einen Schwenkarm 65 und einen Halterarm 64 umfasst. Die Mikroskophalterung 61 kann auch als eine eigenständige Einheit angesehen werden, die nicht Teil des Stativs ist. An der Mikroskophalterung 61 ist ein Operationsmikroskop 3 befestigt. Am Stativ 51 sind außerdem eine Lichtquelle 66 zur Objektbeleuchtung sowie ein Netzanschlussgerät und ein Bedienelement 67 für elektrische Komponenten des Mikroskops 3 und des Stativs 51 angeordnet. Sowohl die Lampen als auch die Versorgungseinheit können Schwingungen generieren, bspw. wenn eine Kühlung dieser Einheiten notwendig ist. Zudem können auch wie bei einem nicht Motor getriebenen Stativ über den Stativfuß 55 und die Räder 56 Schwingungen vom Boden aufgenommen werden. Das Stativ 51 umfasst daher eine aktive Schwingungsdämpfungsvorrichtung mit einem als Beschleunigungssensor ausgebildeten Schwingungsaufnehmer 73, einem Regler 57 sowie zwei Aktuatoren 77a, 77b. Der Regler 75 ist zum Empfang eines Beschleunigungssignals über eine Signalleitung 74 mit dem Beschleunigungssensor 73 und zur Ausgabe von Stellsignalen über zwei Signalleitungen 76a, 76b mit den Aktuatoren 77a, 77b verbunden. Als Aktuatoren sind im vorliegenden Ausführungsbeispiel piezoelektrische-Elemente in Stapelbauweise im Einsatz. Diese Erzeugen bei Anlegen einer geeigneten Spannung ein auf dem Federarm 60 einwirkendes Biegemoment. Bei Anlegen einer Wechselspannung kann so eine Schwingung im Federarm induziert werden. Dabei ermittelt der Regler 75 die Stellesignale für die Aktuatoren 77a, 77b auf der Basis des empfangenen Beschleunigungssignals derart, dass eine Gegenschwingung zu einer detektierten Mikroskopschwingung erzeugt wird, welche die detektierten Mikroskopschwingung im Wege der destruktiven Interferenz dämpft.

In den beschriebenen Ausführungsbeispielen sind piezokeramische Aktuatoren sowie ein Aktuator mit einem magnetischen Masseschwinger und einer elektromagnetischen Anregungseinrichtung zur Anwendung gekommen. Es können jedoch auch andere Aktuatoren zum Einsatz kommen bspw. ferroelektrische Aktuatoren. Ferroelektrische Materialien bilden eine Untergruppe der piezoelektrischen Materialien, die sich zum Herstellen sehr präzise wirkende Aktuatoren einsetzen lässt und sich daher besonders zum Herstellen von Aktuatoren zum Dämpfen von Schwingungen in Stativen eignet.

Die vorliegende Erfindung stellt eine Möglichkeit zur Verfügung, Schwingungen in Stativen für medizinisch-optische Instrumente effektiv aktiv zu dämpfen. Dabei wird mittels Aktuatoren entweder eine mechanische Gegenspannung an der Oberfläche eines Lenkers erzeugt, welche die aus der zu dämpfenden Schwingung resultierende mechanische Spannung kompensiert, oder es wird ein Biegemoment bereitgestellt, welches das aus der zu dämpfenden Schwingung resultierende Biegemoment des Lenkers kompensiert. In beiden Fällen wird der Aktuator von einem Schwingungsbauch der zu dämpfenden Schwingung entfernt an der Oberfläche eines Lenkers angeordnet. Insbesondere ist es vorteilhaft, wenn der Aktuator an oder in der Nähe derjenigen Stelle mit dem höchsten durch die zu dämpfende Schwingung induzierten Biegemoment oder der größten durch die zu dämpfender Schwingung induzierten mechanischen Oberflächenspannung im Träger angeordnet wird.

## Patentansprüche

1. Stativ (1, 51) für ein medizinisch-optisches Instrument (3) mit
- einer Lenkeranordnung (15) mit wenigstens einem Lenker (13, 17, 19, 21, 26, 60), und
- einer aktiven Schwingungsdämpfungsvorrichtung (33, 35, 37, 73, 75, 77, 133, 135, 137, 233, 235, 237), welche wenigstens einen Schwingungsaufnehmer (33, 73, 133, 233) zum Aufnehmen einer zu dämpfenden Schwingung und wenigstens einen Aktuator (37, 77, 137, 237) zum Erzeugen einer dämpfenden Gegenschwingung umfasst,
**dadurch gekennzeichnet, dass**
der wenigstens eine Aktuator (37, 77, 137, 237) von einem Schwingungsbauch der zu dämpfenden Schwingung entfernt an der Oberfläche eines Lenkers (19, 26, 60) der Lenkeranordnung (15) angeordnet ist und zum Ausüben einer Kraft auf den Lenker (19, 26, 60) ausgebildet ist.

2. Stativ (1, 51) nach Anspruch 1, **dadurch gekennzeichnet, dass** der wenigstens eine Aktuator (37, 77, 137, 237) an oder in der Nähe einer Stelle (14) eines Lenkers (19, 26) der Lenkeranordnung (15) angeordnet ist, wo die auf den Lenker (19, 26) einwirkende Last des medizinisch-optischen Instruments (3) die größte mechanische Spannung an der Oberfläche des Lenkers (19, 26, 60) bzw. das größte Biegemoment im Lenker (19, 26, 60) erzeugt.

3. Stativ (51) nach Anspruch 1 oder Anspruch 2, **dadurch gekennzeichnet, dass** wenigstens zwei Aktuatoren (77a, 77b) vorhanden sind, die an zueinander nicht parallelen Oberflächebereichen des Lenkers (60) angeordnet sind.

4. Stativ (1, 51) nach einem der vorangehenden Ansprüche, **dadurch gekennzeichnet, dass** der Lenker (19, 60) der Lenkeranordnung (15) ein Tragarm ist, an dem eine Halterung für das medizinisch-optische Instrument (3) zu befestigen ist.

5. Stativ (1, 51) nach einem der vorangehenden Ansprüche, **dadurch gekennzeichnet, dass** wenigstens ein Aktuator (37, 77, 137) ein piezoelektrisches Material umfasst.

6. Stativ (1, 51) nach Anspruch 5, **dadurch gekennzeichnet, dass** der Aktuator (37, 77, 137) ein piezokeramischer Aktuator ist.

7. Stativ (1, 51) nach Anspruch 5, **dadurch gekennzeichnet, dass** das piezoelektrische Material ein ferroelektrisches Material ist.

8. Stativ (1) nach einem der Ansprüche 1 bis 4, **dadurch gekennzeichnet, dass** wenigstens ein Aktuator (237) einen magnetischen Masseschwinger mit einer magnetisch zum Schwingen anregbaren Masse (240) und eine elektromagnetische Anregungseinrichtung (241) zum Anregen einer Schwingung der Masse (240) umfasst.

9. Stativ (1) nach Anspruch 8, **dadurch gekennzeichnet, dass** die zum Schwingen anregbare Masse des Masseschwinger eine in einem Gefäß (239) befindliche ferromagnetische oder magnetorheologische Flüssigkeit (240) ist.

10. Stativ (1, 51) nach einem der vorangehenden Ansprüche, **dadurch gekennzeichnet, dass** der Schwingungsaufnehmer (33, 73, 237) ein am medizinisch-optischen Instrument (3) oder seiner Halterung zu befestigender Beschleunigungssensor ist.

11. Stativ (1) nach einem der Ansprüche 1 bis 9, **dadurch gekennzeichnet, dass** der Schwingungsaufnehmer (133) ein Biegesensor oder ein Spannungssensor ist, der an oder in der Nähe einer Stelle eines Lenkers (19) der Lenkeranordnung (15) angeordnet ist, an der die auf den Lenker (19) einwirkende Last des medizinisch-optischen Instruments (3) die größte elastische Verformung an erzeugt.

12. Stativanordnung mit einem Stativ (1) nach einem der vorangehenden Ansprüche, einer an der Lenkeranordnung angeordneten Halterung (25) für das medizinisch-optische Instrument (3), und einem an der Halterung (25) befestigten medizinisch-optischen Instrument (3).

13. Stativanordnung nach Anspruch 12, **dadurch gekennzeichnet, dass** das medizinisch-optische Instrument (3) ein Operationsmikroskop ist.
